# EUROPÄISCHE PATENTSCHRIFT

(11) **EP 2 215 104 B1**
(45) Veröffentlichungstag und Bekanntmachung des Hinweises auf die Patenterteilung: **10.04.2013**
(21) Anmeldenummer: 08843298.4
(22) Anmeldetag: 18.10.2008
(51) Int. Cl.: C07J 1/00

(54) **11.BETA.-FLUORO-3-ACETOXYESTRA-3,5-DIEN-17-ON UND VERFAHREN ZU SEINER HERSTELLUNG**
11 -FLUORO-3-ACETOXYESTRA-3,5-DIENE-17-ON AND METHOD FOR THE PRODUCTION THEREOF
11 -FLUORO-3-ACÉTOXYESTRA-3,5-DIÈNE-17-ONE, ET SON PROCÉDÉ DE FABRICATION

(30) Priorität: 24.10.2007 EP 07075924
(43) Veröffentlichungstag der Anmeldung: 11.08.2010
(73) Patentinhaber: Bayer Intellectual Property GmbH, 40789 Monheim (DE)
(72) Erfinder: PETROV, Orlin, 14195 Berlin (DE); SCHNEIDER, Matthias, 14482 Potsdam (DE); BOHLMANN, Rolf, 14055 Berlin (DE); VETTEL, Stephan, 13467 Berlin (DE)
(74) Vertreter: BIP Patents
(86) Internationale Anmeldenummer: PCT/EP2008/008833
(87) Internationale Veröffentlichungsnummer: WO 2009/053008

(56) Entgegenhaltungen:
- WO-A-99/33855
- BENNUA-SKALMOWSKI B ET AL: "A Facile Conversion of Primary or Secondary Alcohols with n-Perfluorobutane-sulfonyl Fluoride/1,8-Diazabicyclo[5.4.0]undec-7-en e into their Corresponding Fluorides" TETRAHEDRON LETTERS, ELSEVIER, AMSTERDAM, Bd. 36, Nr. 15, 10. April 1995 (1995-04-10), Seiten 2611-2614, XP004028262 ISSN: 0040-4039
- E. J. BAILEY ET AL: "A New Method for Replacement of Hydroxyl by Chlorine or Bromine: Preparation of 11.beta.-Halogeno-steroids" CHEMICAL COMMUNICATIONS - CHEMCOM., Nr. 1, 1970, Seiten 106-107, XP002480368 GBROYAL SOCIETY OF CHEMISTRY.

## Beschreibung

Die vorliegende Erfindung betrifft 11β-Fluor-3-acetoxyestra-3,5-dien-17-on (I). 11β-Fluor-3-acetoxyestra-3,5-dien-17-on (1) 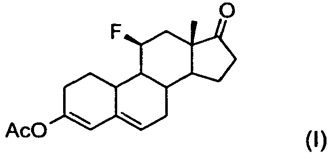 ist als Zwischenstufe für die Herstellung von 11-Fluor-substituierten Steroiden, die als pharmazeutische Wirkstoffe Verwendung finden, geeignet. Des weiteren betrifft die Erfindung ein Verfahren zu dessen Herstellung.

11β-Fluor-7α-substituierte Steroide sind pharmakologisch hochpotente Verbindungen mit einer ausgeprägten androgenen oder antiestrogenen Wirkung. Als Beispiele können die in der WO 2004/011663 und der WO 2002/059139 beschriebenen Androgene, und die in der WO 03/045972, der WO 99/33855 und der WO 98/07740 beschriebenen Antiestrogene angeführt werden.

Ein Schlüsselschritt bei der Synthese dieser Verbindungen ist die Einführung des 11β-FluorSubstituenten durch eine Deoxyfluorierungsreaktion des 11α-Hydroxy-substituierten Vorläufers (WO 2002/059139). Eine etablierte Methode zur direkten Umwandlung von primären und sekundären Alkoholen einschließlich entsprechender Hydroxysteroide in die entsprechenden Fluoride (Deoxyfluorierung) ist in der Literatur beschrieben (z.B. H. Vorbrüggen et al., Tetrahedron Letters, 1995, 2611; J. Yin et al, Organic Letters 2004, 1465; Ch. Marson et al. Synthetic Communications 2002, 2125; US 5,760,255, US 6,248,889). Dabei wird der entsprechende Alkohol mit kommerziell erhältlichem n-Nonafluorbutansulfonylfluorid und einer starken organischen Base (bevorzugt Diazabicycloundecen (DBU)) in einem geeigneten organischen Lösungsmittel (z. B. Toluol, Xylol, Diglyme, Dichlormethan, Hexan, etc.) umgesetzt und nach wässrigem Aufarbeiten, Extraktion und Chromatographie das entsprechende Fluor-Derivat isoliert.

US 6,248,889 beschreibt einen Deoxyfluorierungsprozess, bei dem die Verwendung eines geringen bzw. keines Überschusses an Base als vorteilhaft beschrieben wird.

Vorbrüggen et al. (Tetrahedron Letters, 1995, 2611) beschreiben speziell die Überführung von 11α-Hydroxy-19-norandrost-4-en-3,17-dion (A) in die entsprechende 11β-Fluor-Verbindung durch Umsetzung mit 1.5 Äquivalenten n-Nonafluorbutansulfonylfluorid und 3 Äquivalenten DBU in Toluol bei 24 - 30°C. Nach einer chromatographischen Aufreinigung erhält man 11β-Fluor-19-norandrost-4-en-3,17-dion (B; Beispiel 1 b), c) in WO 2002/059139) in einer Ausbeute von 66 % d. Th..

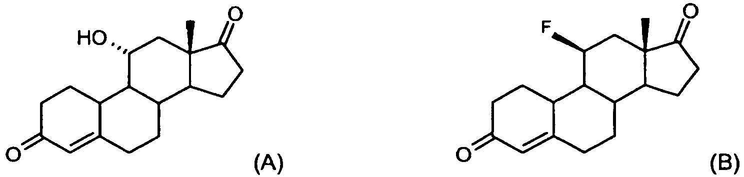

Eine ähnliche Umsetzung (74% Ausbeute nach Chromatographie) mit 1.5 Äquivalenten n-Nonafluorbutansulfonylfluorid und 2.8 Äquivalenten DBU bei 0 °C ist in DE 10104327 beschrieben.

Nachteil dieser Methoden ist neben der mässigen Ausbeute der schlechte Prozessdurchsatz, da große Mengen Lösungsmittel sowohl bei der Reaktion, als auch bei der mehrfachen Extraktion während der Aufarbeitung, erforderlich sind. In der Regel erhält man nach der Reaktionsaufarbeitung ein Rohprodukt, dessen Menge 3 mal größer ist als die Menge des eingesetzten Ausgangsmaterials. Zur Isolierung des Produktes aus dem Rohgemisch ist eine chromatographische Reinigung unumgänglich. Daher sind solche Verfahren zur Herstellung von 11 β-Fluorsteroiden in Multikilogramm-Mengen nicht geeignet.

Aufgabe der vorliegenden Erfindung ist es daher, ein Verfahren zur Herstellung von 11 β-Fluor-3-acetoxyestra-3,5-dien-17-on (1) zu schaffen, welches eine einfache Gewinnung des Produktes gestattet und sich durch eine gute Ausbeute und einen guten Prozessdurchsatz auszeichnet.

Erfindungsgemäß wurde nunmehr gefunden, dass nach der temperaturkontrollierten Reaktion von 11β-Hydroxy-estr-4-en-3,17-dion (A) mit 1,5 - 2 Äquivalenten n-Nonafluorbutansulfonylfluorid in Gegenwart eines Überschusses von mindestens 3,3 Äquivalenten DBU bei - 40 bis - 20°C, gefolgt von einer pH-kontrollierten wässrigen Aufarbeitung und anschliessender Acetylierung des Zwischenproduktes das entstandene 11β-Fluor-3-acetoxyestra-3,5-dien-17-on überraschenderweise spontan aus dem Reaktionsgemisch kristallisiert und in einer sehr hohen Ausbeute (79 - 86% d.Th.) durch einfache Filtration des Reaktionsgemisches gewonnen werden kann.

Als Lösungsmittel für die Deoxyfluorierungsreaktion können aprotische Lösungsmittel wie zum Beispiel Methylenchlorid, Toluol, Ethylacetat, Isopropylacetat oder Benzotrifluorid dienen. Bevorzugt wird Ethylacetat angewendet.

Für den zweiten Prozessschritt (Acetylierungsreaktion) werden Essigsäureanhydrid oder Isopropenylacetat, bzw. Vinylacetat in Gegenwart starker Säuren wie zum Beispiel p-Toluolsulfonsäure (p-TsOH), Methansulfonsäure, Schwefelsäure oder Bromwasserstoff (HBr) verwendet.

Bevorzugt wird Essigsäureanhydrid in Anwesenheit einer katalytischen Menge p-TsOH verwendet.

Das so in guter Ausbeute und Reinheit gewonnene 11 β-Fluor-3-acetoxyestra-3,5-dien-17-on kann durch Hydrolyse in einem, dem Fachmann bekannten Bromierungs-/Dehydrobromierungs-Prozess in das entsprechende 11β-Fluor-19-norandrost-4,6-dien-3,17-dion (WO 2002/059139; Beispiel 1 c) überführt werden.

Dieses wiederum kann nach bekannten Verfahren zu Androgenen oder Antiestrogenen weiter umgesetzt werden.

Die nachfolgenden Beispiele dienen der näheren Erläuterung der Erfindung. Alle Temperaturen sind in Grad Celsius (unkorrigiert) und alle Mengenangaben sind in Gewichtsprozent angegeben, soweit nicht anders vermerkt.

### Beispiele:

### 11-β-Fluor-3-acetoxyestra-3,5-d ien-17-on

**1.** Eine Mischung aus 50 g 11a-Hydroxyestre-4-en-3,17-dion und 85,6 ml DBU (3,3 Äqu.) in 250 ml Ethylacetat wird auf -35°C bis -40°C abgekühlt. Dazu wird eine Lösung von 50 ml n-Nonafluorbutansulfonylfluorid (1,6 Äqu.) in 100 ml Ethylacetat bei -35°C unter starkem Rühren langsam zugetropft. Die Reaktionslösung wird 15 Stunden bei -35°C gerührt, bis laut HPLC der Anteil 11α-Hydroxyestr-4-en-3,17-dion < 1% beträgt. Danach wird auf -10°C erwärmt, 60 min. nachgerührt und die Reaktion mit 20 ml Wasser versetzt.
   Die Kühlung wird entfernt und die Reaktionsmischung mit 100 ml 2N Schwefelsäure versetzt. Es wird 90 min bei 10°C nachgerührt. Die Phasen werden getrennt und die organische Phase wird mit 22 ml 2N Schwefelsäure versetzt, um einen pH-Wert von 2 einzustellen. Die Phasen werden wieder getrennt und die organische Phase wird mit 50 ml ges. NaHCO₃-Lösung und mit 50 ml ges. Kochsalzlösung gewaschen und unter vermindertem Druck auf ca. 160 ml eingeengt. Es wird zweimal mit jeweils 200 ml Ethylacetat versetzt und bei 60°C und vermindertem Druck auf ca. 160 ml eingeengt. Es entsteht jeweils ein rührbarer Kristallbrei.
   Zu dem rührbaren Kristallbrei werden bei 20°C 147,1 ml Essigsäureanhydrid (9 Äqu.) zugegeben. Es wird auf 0°C gekühlt. Innerhalb von 4 h werden in 3 Portionen insgesamt 2,24 ml Methansulfonsäure (0,4 Äqu.) zugegeben Es wird weitere 44 Stunden bei 0°C gerührt und anschliessend das ausgefallene Reaktionsprodukt abgesaugt und viermal mit 40 ml eiskaltem Isopropylacetat gewaschen und bei 40°C im Vakuum getrocknet.
   Man erhält 49,6 g (86,1% d. Th.) 11-β-Fluor-3-acetoxyestra-3,5-dien-17-on als hellbeigen, kristallinen Feststoff. Smp. 175-177 °C; [α]_{D} = - 32.3° in CHCl₃; H-NMR (δ in CHCl₃): 5.82 [1 H, d, *J* = 2 Hz ], 5.50 [1 H, m], 5.08 [1 H, d(br), *J* = 49 Hz], 2.15 [3 H, s], 1.04 [3 H, d, *J* = 1.5 Hz] [ppm].
**2.** 0,5 kg 11α-Hydroxyestr-4-en-3,17-dion und 856 ml DBU (3,3 Äqu.) werden in 3,25 l Essigester suspendiert und auf -35°C bis -40°C abgekühlt. Dazu wird innerhalb von 120 min eine Lösung von 500 ml n-Nonafluorbutansulfonylfluorid (1,6 Äqu.) in 250 ml Ethylacetat zugegeben.

Die Reaktionslösung wird nach beendeter Zugabe 6 Stunden bei -35°C gerührt Danach wird innerhalb von 30 min auf -10°C erwärmt. Man lässt noch 120 min bei -10°C nachrühren und versetzt die Reaktion mit 600 ml 2N Schwefelsäure. Es wird 60 min nachgerührt und dabei auf 30°C erwärmt.

Die Phasen werden getrennt und die organische Phase wird mit 600 ml 2N Schwefelsäure versetzt, um einen pH 3 einzustellen. Die Phasen werden wieder getrennt und die organische Phase wird mit 800 ml ges. Natriumhydrogencarbonatlösung gewaschen und bei 60°C und 120 mbar auf ca. 1,8 I eingeengt. Es wird zweimal mit jeweils 1,5 I Ethylacetat versetzt und bei 60°C und 120 mbar auf ca. 1,8 l eingeengt. Es entsteht jeweils ein rührbarer Kristallbrei der anschliessend mit 1,471 l Essigsäureanhydrid (9 Äqu.) versetzt wird. Das Gemisch wird auf 0°C gekühlt und nach einer Zugabe von 132 g p-Toluolsulfonsäure (0,4 Äqu.) 24 Stunden bei 0°C gerührt. Das ausgefallene Reaktionsprodukt wird abgesaugt, fünfmal mit 500 ml eiskaltem Ethylacetat gewaschen und bei 40°C im Vakuum getrocknet.

Man erhält 475 g (82,5% d. Th.) 11-β-Fluor-3-acetoxyestra-3,5-dien-17-on als hellbeigen, kristallinen Feststoff.

### Hydrolyse von 11β-Fluor-3-acetoxyestra-3,5-dien-17-on zu 11β-Fluor-19-norandrost-4-en-3,17-dion

950 g 11β-Fluor-3-acetoxyestra-3,5-dien-17-on werden in 10 l Methanol gelöst. Dazu werden 2,0 l einer gesättigten Kaliumcarbonat-Lösung bei 50°C zugegeben. Nach 5 Stunden wird das Reaktionsgemisch auf Raumtemperatur abgekühlt und auf ca. 3 l im Vakuum aufkonzentriert. Das ausgefallene Produkt wird abfiltriert, mit Wasser gewaschen und getrocknet. Man erhält 755 g (91% d.Th.) 11β-Fluor-19-norandrost-4-en-3,17-dion als weissen Feststoff. Schmp. 171-173°C.

## Patentansprüche

1. 11β-Fluor-3-acetoxyestra-3,5-dien-17-on.

2. Verfahren zur Herstellung von 11β-Fluor-3-acetoxyestra-3,5-dien-17-on, **dadurch gekennzeichnet, dass** 11α-Hydroxyestr-4-en-3,17-dion mit 1 bis 3 Äquivalenten n-Nonafluorbutansulfonylfluorid und 3 bis 5 Äquivalenten Diazabicycloundecen (DBU) bei -40 bis -20 °C in einem organischen nicht-protischen Lösungsmittel reagieren gelassen und nach einer wässrigen Zwischenaufarbeitung mit 5 bis 10 Äquivalenten eines Acetylierungsmittels in Gegenwart von 0,01 bis 1 Äquivalenten einer starken Säure umgesetzt und das ausgefallene Produkt durch Filtration des Reaktionsgemisches gewonnen wird.

3. Verfahren zur Herstellung von 11β-Fluor-3-acetoxyestra-3,5-dien-17-on nach Anspruch 2, **dadurch gekennzeichnet, dass** mehr als 3 Äquivalente DBU verwendet werden.

4. Verfahren zur Herstellung von 11β-Fluor-3-acetoxyestra-3,5-dien-17-on nach Anspruch 2, **dadurch gekennzeichnet, dass** Ethylacetat als Lösungsmittel verwendet wird.

5. Verfahren zur Herstellung von 11β-Fluor-3-acetoxyestra-3,5-dien-17-on nach Anspruch 2, **dadurch gekennzeichnet, dass** mit Essigsäureanhydrid als Acetylierungsmittel umgesetzt wird.

6. Verfahren zur Herstellung von 11β-Fluor-3-acetoxyestra-3,5-dien-17-on nach Anspruch 2, **dadurch gekennzeichnet, dass** mit p-Toluolsulfonsäure (p-TsOH) als starker Säure umgesetzt wird.

## Claims

1. 11β-Fluoro-3-acetoxyestra-3,5-dien-17-one.

2. Process for preparing 11β-fluoro-3-acetoxyestra-3,5-dien-17-one, **characterized in that** 11α-hydroxyestra-4-ene-3,17-dione is allowed to react with 1 to 3 equivalents of n-nonafluorobutanesulfonyl fluoride and 3 to 5 equivalents of diazabicycloundecene (DBU) at -40 to -20°C in an organic aprotic solvent and, after an aqueous intermediate workup, reacted with 5 to 10 equivalents of acetylating agent in the presence of 0.01 to 1 equivalent of a strong acid, and the precipitated product is obtained by filtering the reaction mixture.

3. Process for preparing 11β-fluoro-3-acetoxyestra-3,5-dien-17-one according to Claim 2, **characterized in that** more than 3 equivalents of DBU are used.

4. Process for preparing 11β-fluoro-3-acetoxyestra-3,5-dien-17-one according to Claim 2, **characterized in that** ethyl acetate is used as the solvent.

5. Process for preparing 11β-fluoro-3-acetoxyestra-3,5-dien-17-one according to Claim 2, **characterized in that** reaction is effected with acetic anhydride as the acetylating agent.

6. Process for preparing 11β-fluoro-3-acetoxyestra-3,5-dien-17-one according to Claim 2, **characterized in that** reaction is effected with p-tolulenesulfonic acid (p-TsOH) as the strong acid.

## Revendications

1. 11β-fluoro-3-acétoxyestra-3,5-dién-17-one.

2. Procédé pour la préparation de 11β-fluoro-3-acétoxyestra-3,5-dién-17-one, **caractérisé en ce qu'**on fait réagir de la 11α-hydroxyestr-4-ène-3,17-dione avec 1 à 3 équivalents de fluorure de n-nonafluorobutane-sulfonyle et 3 à 5 équivalents de diazabicyclo-undécène (DBU) à une température de -40 à -20 °C dans un solvant organique aprotique et après un traitement intermédiaire aqueux on fait réagir avec 5 à 10 équivalents d'un agent d'acétylation en présence de 0,01 à 1 équivalent d'un acide fort et on obtient par filtration du mélange réactionnel le produit précipité.

3. Procédé pour la préparation de 11β-fluoro-3-acétoxyestra-3,5-dién-17-one selon la revendication 2, **caractérisé en ce qu'**on utilise plus de 3 équivalents de DBU.

4. Procédé pour la préparation de 11β-fluoro-3-acétoxyestra-3,5-dién-17-one selon la revendication 2, **caractérisé en ce qu'**on utilise comme solvant de l'acétate d'éthyle.

5. Procédé pour la préparation de 11β-fluoro-3-acétoxyestra-3,5-dién-17-one selon la revendication 2, **caractérisé en ce qu'**on fait réagir avec de l'anhydride acétique en tant qu'agent d'acétylation.

6. Procédé pour la préparation de 11β-fluoro-3-acétoxyestra-3,5-dién-17-one selon la revendication 2, **caractérisé en ce qu'**on fait réagir avec de l'acide p-toluènesulfonique (p-TsOH) en tant qu'acide fort.
